(19) Europäisches Patentamt
European Patent Office
Office européen des brevets

(11) Publication number: **0 047 596**
**B1**

(12) **EUROPEAN PATENT SPECIFICATION**

(45) Date of publication of patent specification: **30.11.83**

(21) Application number: **81303777.7**

(22) Date of filing: **19.08.81**

(51) Int. Cl.[3]: **C 07 C 1/04**, C 07 C 29/15, C 07 C 41/09, C 07 C 45/49, C 07 C 51/10, C 01 B 3/34

# (54) Synthesis for producing carbon compounds from a carbon oxide/hydrogen synthesis gas.

(30) Priority: **04.09.80 GB 8028568**
**22.01.81 GB 8101950**

(43) Date of publication of application:
**17.03.82 Bulletin 82/11**

(45) Publication of the grant of the patent:
**30.11.83 Bulletin 83/48**

(84) Designated Contracting States:
**BE DE FR GB IT NL**

(56) References cited:
DE - B - 2 427 330
FR - A - 2 211 437
FR - A - 2 396 068
GB - A - 2 022 074
US - A - 4 235 044

(73) Proprietor: **IMPERIAL CHEMICAL INDUSTRIES PLC**
**Imperial Chemical House Millbank**
**London SW1P 3JF (GB)**

(72) Inventor: **Pinto, Alwyn**
**18 Cambridge Road**
**Linthorpe Middlesbrough Cleveland (GB)**

(74) Representative: **Chapman, Kenneth Hazel et al,**
**Imperial Chemical Industries PLC Legal Department: Patents Thames House North Millbank**
**London SW1P 4QG (GB)**

**EP 0 047 596 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Synthesis for producing carbon compounds from a carbon oxide/hydrogen synthesis gas

This invention relates to a process of synthesis for producing carbon compounds from a carbon oxide/hydrogen synthesis gas.

Such a synthesis is typified by the production of methanol from a gas made by catalytic reaction of a gaseous or vaporisable hydrocarbon feedstock with steam. When such feedstocks contain at least 2 hydrogen atoms per carbon atom, the gas contains at least sufficient hydrogen for methanol synthesis and can be passed to the synthesis with no loss of pressure due to chemical treatment. It has been proposed to produce methanol from other feedstocks, such as natural gases containing much carbon dioxide and especially from heavier hydrocarbons, coal, coke or shale, which are deficient in hydrogen and also have to be gasified by partial oxidation. Then the raw synthesis gas is subjected to steps a catalytic shift reaction and carbon dioxide removal in order to correct the composition of the gas. Such steps are complicated and thermally not very efficient. Moreover, they introduce a substantial pressure-drop, as a result of which either the initial partial oxidation has to be operated at an uneconomically high pressure or else an undesirably high consumption of energy in gas compression is incurred. Examples of the shift reaction applied to carbon monoxide made by partial oxidation are to be found in UK 770765, UK 1309872 and the article by Staege in Erdöl-Erdgas-Zeitschrift 1976, *92*, 381—387.

We have now devised a process in which some or all of the above defects can be avoided, in methanol production and in other syntheses. It is also capable of dealing with hydrocarbons encountered in the synthesis gas or as by-products in the process.

According to the invention a process for producing one or more carbon compounds by reaction of carbon monoxide (CO) with hydrogen comprises the steps

I. providing a starting gas containing CO but deficient in hydrogen;
II. processing it to produce a gas having the required hydrogen to CO ratio; and
III. reacting the resulting gas incompletely over a catalyst to synthesise the said carbon compounds or an intermediate therefor;

and is characterised by

(i) producing the gas having the required hydrogen to CO ratio by mixing a hydrogen rich gas with the starting gas; and
(ii) producing the hydrogen rich gas by subjecting to the shift reaction and/or to the steam/hydrocarbon reaction and/or to carbon dioxide removal a mixture of the starting gas with either or both of (a) the product of such subjecting and (b) unreacted gas after separation of synthesis products in step III.

As a result of feature (i) starting gas can pass to step III without incurring the pressure drop due to reaction and/or carbon dioxide removal steps. As a result of feature (ii) the shift step can be carried out in advantageous ways.

In the ensuing description a number of process steps will be referred to by proper means. These names are Registered Trade Marks or otherwise proprietary to companies making design data available under licence.

The starting gas can be derived from catalytic reforming of carbon dioxide rich natural gas or catalytic partial oxidation of hydrocarbons boiling at not over 200°C. It may be a by-product such as the off-gas from a basic oxygen steelmaking furnace. More conveniently it is derived from partial oxidation of a carbonaceous feedstock such as a gaseous hydrocarbon, volatilisable hydrocarbon, heavy oil such as crude oil or residual oil, solids such as coal, coke and shales and waste materials such as polymers and wood products. The pressure of this gas is preferably at least 10 bar abs. especially in the range 20—60 bar abs. and thus the gas can be provided by a partial oxidation step at a pressure high enough not to need compression. Suitable steps are those known by the names of Shell, Texaco, Lurgi and Shell-Koppers. Since, however, the process can minimise pressure losses, the gas can be provided by a partial oxidation at pressure under 10 bar abs. such as the Koppers-Totzek and Winkler processes, followed by compression to the preferred level.

The starting gas suitably contains 20—80% v/v of CO on a dry and carbon dioxide ($CO_2$)-free basis. As derived from partial oxidation it commonly contains 10—50% v/v of hydrogen and up to 15, especially up to 10% v/v of methane, nitrogen and noble gases. The invention is especially valuable when the said CO content is over 50, especially over 60% v/v, as for example in gas derived from partial oxidation of coal by the Koppers-Totzek or Shell-Koppers process. The effect of carrying out step II in the presence of the added gas is to bring the CO content down to a level, especially under 45% v/v, at which simplifications can be made, for example use of a simple adiabatic reactor or combining shift with other reaction steps or providing an adequate steam to CO level at a low steam to gas ratio, and good heat recovery can be obtained.

The starting gas has usually been purified as follows, unless it has been produced by catalytic reforming:

removal of particles such as of dust, tar and carbon, usually by water-scrubbing and often with transfer of carbon to a hydrocarbon for recycle to the partial oxidation;

Scrubbing with an absorbent liquid to remove $CO_2$, $H_2S$ and possibly other sulphur compounds such as COS.

There may also be a step of removing nitrogen oxides and HCN by catalytic or other treatment, and purifications other than by scrubbing could be used.

For the scrubbing, either of the two available classes of process can be used. In one class $CO_2$, $H_2S$ and COS can be thoroughly removed. Until recently only the "Rectisol" process, using methanol as absorbent, was capable of this. "Rectisol" requires the gas to be cooled to −10° to −40°C, which usually entails an initial stage of methanol treatment to remove water before the cold removal of sulphur compounds. Other processes using for example absorbents based on tetramethylene sulphone, are available or under development. The other class, using absorbents such as the chemical and physical solvents (other than methanol) set out below, can remove $CO_2$ and $H_2S$ efficiently but not the refractory sulphur compounds. They have the advantage of being operable at temperatures in the range −10 to 100°C, but the refractory sulphur compounds have to be decomposed in a separate stage with formation of $H_2S$, which is then removed as part of the purification before step I.

For the absorbent-scrubbing the pressure of the gas is preferably at the level indicated above. Apart from possibly a precautionary $H_2S$-absorbent bed such as zinc oxide or a molecular sieve, the gas passes to the synthesis with substantially no further purification.

If the processing to produce a gas rich in hydrogen involves a shift stage this may be a separate step or may be part of another step. Thus if the synthesis is of methanol from a mixture of CO, $CO_2$ and hydrogen as normally practised, little if any shift reaction takes place during synthesis and thus the unreacted gas must be subjected to the shift reaction in a separate step and then to $CO_2$ removal. If, however, methanol synthesis is carried out in presence of steam, possibly instead of $CO_2$ initially fed or possibly at a higher concentration than this, then shift will accompany synthesis and the unreacted gas will require $CO_2$ removal but less, possibly no, separate shift reaction. Likewise if the synthesis in itself or in accompanying reactions produce water, this reacts with CO over the synthesis catalyst and decreases or removes the need for a separate shift reaction. Further details are given below.

When methanol synthesis is accompanied by shift reaction by adding steam to the synthesis gas about to enter the synthesis catalyst, the shift reaction can occur over a catalyst particularly formulated to withstand shift reaction conditions and possibly having only low if any methanol synthesis activity. The shift step can be carried out in a separate reactor or in the upstream-most bed or beds of a synthesis reactor or in an inlet portion of a bed containing shift catalyst followed by synthesis catalyst. Preferably the shift reaction take place over a copper-containing catalyst, and as a result the shift outlet gas requires little or no adjustment of temperature before it enters the synthesis. Typically the stream to dry gas ratio of the gas is under 0.2 entering the shift step and under 0.02 leaving it, when the synthesis is of methanol.

The shift reaction may accompany a hydrocarbon/steam reaction. If the gas processed to give the hydrogen rich stream contains a hydrocarbon (which term includes a hydrocarbon derivative), it is processed preferably by reaction with steam over a catalyst in conditions such that it is converted to carbon oxides and hydrogen. Such conditions include preferably a temperature over 550°C, especially in the range 600—900°C and a steam to hydrocarbon carbon molar ratio of at least 2, especially in the range 3—10. Since the steam/hydrocarbon reaction is endothermic, the necessary heat can be provided for example by one or more times heating the steam/gas mixture and passing it over a catalyst, or by external heating (the catalyst being contained in tubes supported in a furnace) or by internal heating by added oxygen, in any event aided by any simultaneous shift reaction. The catalyst is typically one or more metals from Group VIII of the Periodic Table, especially nickel or cobalt, on a refractory support. Depending on the composition of the gas processed in this step and of hydrogen content required, the hydrocarbon/steam reaction step can provide enough hydrogen to make shift reaction unnecessary or can itself effect sufficient shift reaction or can be followed by one or more steps of shift reaction.

The said hydrocarbon can enter in from various sources, especially the following:

(i) from a gasification step, such as the Lurgi process, operated in conditions such that methane is present in the raw gas. Those conditions include especially a temperature in the range 900—1700°C and/or a pressure in the range 10—120 bar abs. The methane content is typically in the range 1—20, especially 4—10% v/v on a dry basis. As a result of removal of CO and $H_2$ by methanol synthesis and of $CO_2$ removal steps, the methane content of the gas processed to give the hydrogen-rich gas is typically higher than this by a factor of 1.5 to 3;

(ii) as by-products of the synthesis of methanol, dimethyl ether, higher alcohols, mixed hydrocarbons and/or oxygenated hydrocarbons or OXO or carbonylation products, or of aromatisation especially of methanol or dimethyl ether; or

(iii) as recovered off-gases from let-down vessels or distillation columns or saturators fed with water containing hydrocarbons.

The hydrocarbon content, calculated as equivalent methane, after such processing is typically less than 5, especially under 1% v/v on a dry basis.

If the gasification step produces sufficient methane and/or the synthesis produces sufficient by-product hydrocarbons or hydrogen, the ratio to CO of hydrogen and potential hydrogen in the gas to be processed to give the hydrogen-rich stream can be high enough to make $CO_2$ removal unnecessary. By "potential hydrogen" is meant the hydrogen obtainable by the reactions:

$$C_nH_{2n+2} + nH_2O \rightarrow nCO + (2n+1)H_2$$

and

$$CO + H_2O \rightarrow CO_2 + H_2$$

to the extent to which they occur. A particular example is a process involving synthesis of methanol and/or dimethyl ether (which contain 2 hydrogen atoms per carbon atom) followed by aromatisation, the liquid products of which contain less than 2 hydrogen atoms per carbon atom and the gaseous products more than 2.

If a separate shift step is used, it is most conveniently at an outlet temperature in the range 300—550°C and over an iron-chrome catalyst, owing to the still relatively high CO content of the gas being treated. If the gas being treated contains compounds, such as methanol, that would form methane over such a catalyst, a zinc-chrome catalyst can be used. If desired, a second stage, of low temperature shift (200—260°C) over a copper-containing catalyst, can follow the stage at 300—450°C. Such a catalyst does not cause methane formation. By low temperature shift an outlet CO content in the range 0.2 to 3.0% can be readily attained and an export hydrogen stream produced, as described below. Steam for the shift reaction is provided preferably by humidification with hot water from heat-exchange with reacted gas in the synthesis step. In this event the high grade heat in the gas leaving shift at 300—550°C is available for high pressure (40—140 bar) steam generation and the effect is to upgrade the heat recovered from the synthesis.

In order to carry out a shift stage in an adiabetic bed, with minimum steam consumption and maximum heat recovery, the conditions of initial CO concentration in the gas mixture, steam to gas ratio and temperature are chosen preferably to give an outlet CO concentration in the range 3 to 18% v/v on a dry basis, before $CO_2$ removal. Concentrations of CO of over 10% v/v are preferred, if the synthesis can be operated with a correspondingly low ratio of hydrogen to carbon oxides. Thus a starting steam to total gas ratio in the range 0.4 to 0.6 and an outlet temperature over 400°C are very suitable.

A separate shift step can be simplified by limiting the extent of shift to the minimum that will provide hydrogen for the synthesis. It can thus be advantageous overall to operate the synthesis at what would normally be regarded as a deficiency of hydrogen. In particular, for methanol synthesis, whereas it has been customary to operate with

$$R = \frac{H_2 - CO_2}{CO + CO_2} = \text{at least 2, possibly up to 15 or more,}$$

in the present process R is preferably in the range 0.8 to 2.0, especially 1.0 to 1.8. For methanol synthesis in absence of added steam, since the synthesis reaction removes carbon oxides and hydrogen on the basis of $R = 2$, the unreacted gas after separation of methanol is still richer in CO and it is necessary to use shift but this can be incomplete and a simple adiabatic bed can be used. If steam is added to the gases entering methanol synthesis the value of R does not change but owing to the shift reaction the ratio $H_2/CO$ increases and if it exceeds 2.0 the unreacted gas is (after $CO_2$ removal) enriched in hydrogen. A steam addition sufficient to produce an $H_2/CO$ ratio in the range 2.5 to 5.0 provides a suitable balance of methanol synthesis and shift and can make separate shift unnecessary. The intermediate case of separate shift to give an $H_2/CO$ ratio of 0.8 to 2.0, especially 1.0 to 1.8, and further shift by adding steam to the synthesis gas is preferred when the CO content of the starting gas is over 50% v/v.

In the $CO_2$ removal step the so-called "chemical" solvents can be used, such as ethanolamines or potassium carbonate, especially in the established processes such as "Amine Guard", "Benfield", "Benfield-DEA", "Vetrocoke" and "Catacarb", at any of the pressures contemplated for the process of the process of the invention.

For effective use of physical solvents the process pressure is preferably at least 20 bar abs.; however, since synthesis gas to be used over a copper-containing catalyst preferably contains 1—15, especially 2—10% v/v of carbon dioxide, the pressure need not be as high as in the production of ammonia synthesis gas in which substantially complete removal of carbon dioxide is needed. Provided enough hydrogen is present, excess $CO_2$ can be removed by reverse shift reaction accompanying synthesis.

As examples of physical solvents there may be mentioned

Tetramethylene sulfone ("Sulfinol")
propylene carbonate (Fluor)
N-methyl-2-pyrrolidone ("Purisol")
dimethyl ether of polyethyleneglycol ("Selexol")
methanol ("Rectisol")

The process is not, however, limited to solvent methods of $CO_2$-removal, as will be described below.

In any embodiment of the process in which $CO_2$ removal is practised, this step can be a separate one or can be the same as that used in step I. If desired such a separate step can remove part of the $CO_2$ and the step I $CO_2$ removal can remove further $CO_2$. If two $CO_2$-removal steps are used, they can be linked to a common regenerator, if the absorbent is the same.

Whether or not step II includes a separate shift step, it preferably includes production of a hydrogen stream by a physical method such as cryo-genic fractionation, selective adsorption or membrane diffusion. Preferably the gas in step II is divided into 2 streams, one of which is purified by solvent $CO_2$-removal, the other by a physical method. If there is shift, it preferably precedes such division. The physical treatment step makes it possible to increase the purity of the hydrogen-enriched gas without the complication of low temperature shift. It also provides a hydrogen stream that can be exported, for use in for example ammonia synthesis: indeed the invention includes such an integrated process for production of methanol and ammonia. A more complete shift reaction is preferable when hydrogen is to be exported. The outlet CO content, preferably in the range 0.2 to 3.0% v/v on a dry basis, can be achieved by multi-stage high temperature shift with removal of steam and $CO_2$ between stages. Preferably it is achieved by high temperature shift followed by low temperature shift, in which event there need be only cooling between the stages. The shifted gas is then cooled to below the dewpoint of steam, separated from condensed water and subjected to $CO_2$ removal. The resulting gas is pure enough to provide the hydrogen-rich stream to be mixed with the hydrogen-deficient gas as previously described. It is also suitable for conversion to ammonia synthesis gas and, in a process in which the starting gas is provided by gasifying a feedstock with oxygen derived from air separation, it is mixed with nitrogen available from the air separation. Preferably this is done by washing the $CO_2$-removed gas with liquid nitrogen, since this affords a very pure synthesis gas.

The physical separation is also highly valuable for removing non-reactive gases from the synthesis gas. Since most sources of CO produce also small or fractional percentages of nitrogen and/or methane, and since many synthesis processes produce such percentages of methane, such non-reacting gases slowly build up if the synthesis process involves recycle; thus a purge of synthesis gas has to be maintained. In synthesis processes as usually operated a stream of recycle gas after product separation is purged and proposals have been made to treat it for recovery of carbon oxides and/or hydrogen. In the process of the invention the physical treatment in step (II) is the most convenient way of removing the non-reacting gases.

Two particular process sequences for carrying out the invention should be mentioned.

A preferred form of the process comprises mixing a gas rich in CO with a gas rich in hydrogen and a synthesis recycle gas, compressing the mixture, dividing the compressed mixture into two streams, passing one stream to steps to produce the gas rich in hydrogen and passing the other stream to synthesis to produce a liquid synthesis product and the recycle gas. For such a process compression by up to 50% or even up to 20% suffices, as in a synthesis circulating pump, and acts upon both the hydrogen stream and the synthesis stream.

An alternative sequence applicable to the invention in another aspect described below comprises mixing a gas rich in hydrogen and a synthesis recycle gas, compressing the mixture, passing the compressed mixture to synthesis to produce a liquid synthesis product and an unreacted gas, dividing the unreacted gas into two streams, passing one stream to steps to produce the gas rich in hydrogen and passing the other stream to the mixing point as the synthesis recycle gas. This form of the process requires more compression because the pressure of the gas entering such steps is lower to the extent of the pressure drop through the synthesis. If desired, an additional compressor for the hydrogen rich gas production circuit could be used.

In any process in which the starting gas has been compressed, the hydrogen rich gas or unreacted gas can be fed into the starting gas upstream of the compressor used.

If the synthesis is to be of methanol, any suitable synthesis process can be used. Thus synthesis may be over a catalyst in tubes surrounded by a coolant or in the space around tubes containing coolant. The coolant may be for example pressurised water or a mixture of diphenyl and diphenyl ether; the pressurised water can be used as feed for a boiler or humidifier or, like the mixture, heat-exchanged in liquid form with suitable water to be fed to a boiler or humidifier. Alternatively the coolant water may be allowed to boil and the resulting intermediate pressure steam used as process feed or in an engine or condensed in indirect or direct heat exchange with pressurised water. In a second process the catalyst temperature can be controlled by heat exchange with cool feed gas passing through tubes in the catalyst bed or through the space surrounding catalyst-filled tubes. In a third process the catalyst bed can be in several parts with heat-abstraction by indirect heat exchange between the parts. Each part of the bed operates adiabatically and thus the construction of the reactor is simpler than for the first or second process. In a fourth, widely used, process, the temperature is controlled by injecting cool synthesis gas ("quench gas") into the hot reacting synthesis gas. Quench gas can be injected into mixing chambers between successive parts of a catalyst bed or successive reactor vessels. A very convenient system involves a single body of catalyst in which are disposed catalyst-free perforated hollow bars each having a sparger for introducing the quench gas, the bars being large enough in cross

section for their interiors to constitute mixing zones and close enough together or to the catalyst bed walls to cause a substantial proportion of reaction mixture to pass through their interiors, as described in our UK specification 1,105,614. The temperature of quench gas can be below 50°C, but thermal efficiency is better if it is at between 50 and 150°C. A composite reactor having quench cooling at 2—4 upstream levels and indirect heat exchange before the downstream-most bed also has advantages.

The volume space velocity of the flow of gas through the synthesis catalyst bed is typically in the range 5000—50000 $hour^{-1}$ and is preferably fixed at a level such that the gas leaves the catalyst bed when the quantity of methanol formed has been sufficient to raise the gas temperature to the design level, which is under 300°C and most preferably under 280°C. The methanol content of the reacted gas is preferably 2—8% and thus the pressure is preferably in the range 20—50, especially 35—45 bar abs. By operating at such a relatively low pressure the methanol production rate can be kept at a level such that the exothermic heat of synthesis is taken up by the gas (including quench gas) and need not be removed by indirect heat exchange in the synthesis reactor. Consequently simple reactors having one or more adiabatic catalyst beds such as those described in UK 1105614 can be used, instead of the steam-raising tubular reactors previously disclosed for using synthesis gas made by partial oxidation. Broadly speaking, the synthesis pressure is chosen such that the partial pressures of stoichiometric carbon oxides and hydrogen are of the same order as in synthesis gas made by steam/hydrocarbon reforming. Since the gas fed to the synthesis catalyst in the process of the invention typically contains less than 10% v/v of methane, nitrogen and noble gases and not more than stoichiometric hydrogen, unlike methane steam reforming gas (typically 10—25% $CH_4$, 20—40% excess $H_2$), the total pressure required is typically 50 to 80% of the corresponding process based on steam reforming. The invention thus makes possible a very attractive methanol production process based on partial oxidation, even when the partial oxidation step is at a low pressure and has to be followed by compression.

According to a second aspect of the invention a process for producing methanol by reaction of CO with hydrogen comprises the steps:

I providing a starting gas containing CO but deficient in hydrogen;

II processing it to produce a gas having the required hydrogen to CO ratio; and

III reacting the resulting gas incompletely over a copper containing catalyst to synthesis methanol;

and is characterised by:

i) producing the gas having the required hydrogen to CO ratio by mixing a hydrogen-rich gas with the starting gas;

ii) producing the hydrogen rich gas by subjecting to the shift reaction and/or to the steam/hydrocarbon reaction and/or to $CO_2$ removal one or a mixture of the following gases (a) a side stream of starting gas and (b) unreacted gas after separation of methanol in step III, and removing methane, noble gases and nitrogen from the gas to the extent necessary to attain less than 10% v/v of such gases in the gas to be fed to step III; and

iii) operating step III at a pressure in the range 20—50 bar abs, at a temperature under 300°C and at a space velocity in the range 5000—50000 $hour^{-1}$ and controlling these conditions to give methanol content of the reacted gas in the range 2—8% v/v, whereby the exothermic heat of synthesis is taken up by the gas in one or more adiabatic catalyst beds.

An effect of the step of processing to a hydrogen rich stream is that methanol synthesis can be operated at a considerably lower ratio of recycled gas to fresh gas than has been usual, for example 1.5 to 3.0 instead of 4 to 6. Another effect is that the percentage purge from the gas in the synthesis section can be much higher, for example 15 to 30%, than has been usual (2 to 10%); however, the bulk of the CO and hydrogen in this purge is recovered.

For methanol synthesis the catalyst contains typically metallic copper as its active constituent, with an oxidic support material. The support usually contains also zinc oxide and/or one or more further oxides such as of chromium or metals from Group II—IV of the Periodic Table (especially aluminium) and/or possibly, silver or oxides of boron, rare earth metals, vanadium or manganese. Catalysts not containing copper can be used, but are not preferred because a higher synthesis pressure and temperature are needed.

Other syntheses to which the invention is applicable include:

(a) dimethyl ether, in which event the catalyst is similar to methanol synthesis catalyst but is associated with a dehydrating agent such as alumina;

(b) higher alcohols, in which event the catalyst may be similar to methanol synthesis catalyst but usually contains alkali metal oxide or manganese oxide or both;

(c) mixed hydrocarbons and/or oxygenated hydrocarbons, in which event catalysts based on iron or cobalt or ruthenium or other metals are used, as in the Fischer-Tropsch, Synthol and other processes;

(d) "OXO" or carbonylation process, for making aldehydes or carboxylic acids, esters or anhydrides, in which the catalyst may be heterogeneous or homogeneous; and

(e) methanol synthesis or any of (a) to (d) in combination with a subsequent step of aromatisation, olefin-formation over a zeolite or etherification.

These all produce by-product water and are therefore capable of producing unreacted gas of low CO-content, so that in the processing to a hydrogen-rich stream little or no separate shifting may be needed.

One effect of processing the CO-containing gas in the presence of an added gas is to make it possible to carry out a shift step at a relatively high steam to CO ratio but a relatively low steam to total dry gas ratio. The same applies to the steam/hydrocarbon reaction. As a result most or all the required steam can be introduced by contacting with hot water and little if any need be introduced as steam. Thermal efficiency is therefore improved and the expense of steam generation and water purification is decreased. The following Table 1 illustrates the effect. At the inlet of shift a steam to CO ratio of at least 1.0 (especially at least 1.5) and a steam to dry gas ratio under 0.6 (especially in the range 0.2 to 0.5) can be considered typical for the process of the invention when the shift step is not in the same cycle as synthesis.

For providing such steam the hot water is preferably a waste water stream from the process, particularly a condensate after shift but before $CO_2$ removal, methanol distillation bottoms or purge, synthesis by-products or aromatisation by-products. Such waste water contains dissolved or suspended impurities, but by the direct contacting these are returned to the process and converted to wanted products or recovered in a form in which they can be used as fuel. As a result, discharge of large volumes environmentally objectionable effluents can be avoided. It is already known to return to the feedstock partial oxidation effluents such as carbon black, tar and phenolic liquors, if these are formed.

The hot water for the direct contacting is provided preferably by direct contacting with hot steam-containing gases, for examples as produced by the shift or steam hydrocarbon reaction and/or by indirect heat exchange with heated synthesis gas.

Preferred forms of the invention are represented as flowsheets in the accompanying drawings, in which

Figure 1 shows a methanol production process in which a hydrogen rich gas is produced by applying shift and $CO_2$ removal in a side stream to a mixture of three gases;

TABLE 1

| | Conventional | | | | According to invention | | | |
|---|---|---|---|---|---|---|---|---|
| | Inlet | | Outlet | | Inlet | | Outlet | |
| | kg mol $h^{-1}$ | % v/v dry | kg mol $h^{-1}$ | % v/v dry | kg mol $h^{-1}$ | % v/v dry | kg mol $h^{-1}$ | % v/v dry |
| CO | 7542 | 58.79 | 3500 | 20.73 | 6489 | 20.49 | 2447 | 6.85 |
| $CO_2$ | 1561 | 12.17 | 5602 | 33.19 | 2563 | 8.09 | 6604 | 18.49 |
| $H_2$ | 3512 | 27.38 | 7553 | 44.75 | 18367 | 58.00 | 22409 | 62.73 |
| $H_2O$ | 18 | 0.14 | 29 | 0.17 | 41 | 0.13 | 56 | 0.16 |
| $N_2$ | 195 | 1.52 | 195 | 1.16 | 4208 | 13.29 | 4208 | 11.78 |
| Total | 12828 | – | 16884 | – | 31667 | – | 35723 | – |
| Steam added* | 9300 | – | 5258 | – | 11807 | – | 7765 | – |
| Steam/CO v/v | 1.23 | – | 1.5 | – | 1.82 | – | 3.17 | – |
| Steam/dry gas v/v | 0.725$^+$ | – | 0.311 | – | 0.373 | – | 0.22 | – |

* The figures for "steam added" are additional to those for "$H_2O$", which represent residual water vapour in the gas respectively before steam addition or after steam removal.

$^+$Of the ratio 0.725, 0.413 is added by contact with hot water, 0.312 by feeding steam.

Figure 2 shows a methanol production process in which shift is in the same cycle as methanol synthesis reactor but $CO_2$ removal is in a different cycle; and

Figure 3 shows the methanol synthesis section of an integrated process for producing methanol and ammonia. This illustrates the second aspect of the invention.

In the process of Figure 1 gasifier 10 reacts powdered coal with oxygen and a small quantity of steam to give a gas containing CO and some hydrogen. Thus gas is freed from carbon, dust, HCN and nitrogen oxides in coarse purification treatments, compressed if necessary and then freed of $H_2S$, COS and $CO_2$ by contact with cold methanol. All these are generally indicated by purifier 12. The purified CO-rich gas is united at 14 with a hydrogen-rich stream to be described, then at 16 with a recycle gas stream to be described. The total mixture now has the design R value for methanol synthesis and is fed to the inlet of circulating pump 18, which increases its pressure by 10%. The compressed gas is divided at 20 into a synthesis stream and a shift stream. The synthesis stream is passed to synthesis reactor 22, which for simplicity is shown with a single catalyst bed but in practice may include multiple beds and various cooling means as set out above: it need not include internal indirect heat exchange with a coolant. Incomplete reaction to methanol takes place. The gas leaving reactor 22 is cooled at 24, which indicates generally preheating feed gas, heat recovery (with production preferably of hot water) and final cooling to below the dewpoint of methanol. Methanol is separated from the cooled gas in catchpot 26 and run off at 28 to distillation. Unreacted gas passing overhead from 26 is the recycle stream at point 16.

The shift stream from point 20 is warmed and humidified in packed tower 30 by hot water (heated at least partly at 24) fed in at 32, then fed to shift reactor 36, via a feed/effluent heat exchanger (part of 38, not shown separately). The resulting hot shifted gas is cooled in heat exchanger 38, which includes also high grade heat recovery as steam, boiler feed water heating and cooling to below the dewpoint of steam. Water is separated in catchpot 40 and run off at 42 to be reheated and fed again to tower 30, together with added water. The water depleted gas is divided at 50 into dry-treatment stream (which is passed through pressure-swing adsorption unit 52 to remove $CH_4$, CO, $N_2$ and $CO_2$ at 53 and provide a pure hydrogen stream) and a wet treatment stream. The latter is contacted in tower 44 with a $CO_2$ absorbent solution fed in at 48. The charged solution is run off at 46 to a regenerator (not shown) and returned at 48. The overhead gas is united at 54 with the pure hydrogen, to form the hydrogen-rich stream at point 14.

Table 2 shows typical gas compositions and flow rates in the process, for producing 2500 metric tons of methanol per day.

TABLE 2

| Position | Temp °C | Pres. bar abs | Gas composition % v/v | | | | | | Flow rate kg mol $h^{-1}$ |
|---|---|---|---|---|---|---|---|---|---|
| | | | CO | $CO_2$ | $H_2$ | $CH_4$+$N_2$ | $CH_3OH$ | $H_2O$ | |
| 12 outlet | 35 | 35 | 67.05 | – | 31.22 | 1.74 | – | – | 11249 |
| 18 outlet | 38 | 38.5 | 40.24 | 1.45 | 51.59 | 6.20 | 0.52 | – | 78252 |
| 22 inlet | 240 | 38 | 40.24 | 1.45 | 51.59 | 6.20 | 0.52 | – | 62602 |
| 22 outlet | 270 | 36 | 39.07 | 1.60 | 45.59 | 6.96 | 6.75 | 0.03 | 55727 |
| 26 overhead | 35 | 35 | 41.59 | 1.69 | 48.54 | 7.41 | 0.77 | – | 52321 |
| 28 | 35 | 35 | 0.31 | 0.18 | 0.28 | 0.04 | 98.68 | 0.51 | 3406 |
| 36 outlet | 492 | 36.5 | 11.58 | 16.73 | 50.77 | 4.21 | 0.35 | 16.36 | 23050 |
| 44 outlet | 35 | 36 | 17.04 | 2.00 | 74.73 | 6.19 | 0.04 | – | 12529 |
| 52 outlet to 54 | 35 | 36 | – | – | 100 | – | – | – | 2107 |

In Figure 2 gasifier 10 reacts powdered coal with oxygen and a small quantity of steam to give a gas containing CO and some $CO_2$ and hydrogen. This gas is freed of carbon, dust, HCN and nitrogen oxides in coarse purification treatments, then freed of $H_2S$, COS and $CO_2$ by contact with cold methanol. A recycle stream to be described may be received at 151 after coarse purification but before $CO_2$ removal. These steps are indicated generally by purifier 12. The purified CO-rich gas is united at

114 with a first hydrogen-rich stream to be described, then at 115 with a second such stream which is instead of or in addition to the stream received at 151. The total mixture now has an $H_2$:CO ratio lower than it is intended to use in methanol synthesis (unlike the corresponding mixture in the process of Table 2). It is fed to the inlet of circulating pump 118, which increases its pressure by about 10%. The compressed gas receives an addition of water vapour in packed tower saturator 119 in contact with hot water, which can include waste water from distillative purification of methanol and has been heated in a heater 123 or 124 to be described. The moist gas is then heated in heat exchanger 121 (by heat exchange with reacted methanol synthesis gas) to low temperature shift inlet temperature and passed into bed A of reactor 122, which bed contains low temperature shift catalyst. In bed A the shift reaction brings the $H_2$:CO ratio to the design level but little if any methanol is synthesised. The gas leaving bed A is partly drawn off and cooled in heat recovery 123 (see below), partly passed through bed B, which is charged with methanol synthesis catalyst. Cooled gas from 123 is fed back partly into reactor 122 after bed B as a quench to lower the temperature, which has risen as the gas passes through bed B as a result of the exothermic methanol synthesis. The remainder of the cooled gas is fed back into reactor 122 after methanol synthesis bed C, again as a quench. The temperature rises yet again as the quenched gas passes through methanol synthesis bed D, but is then cooled by indirect heat exchange with shift inlet gas at 121. The cooled gas is reacted further in methanol synthesis bed E and led out to heat recovery and cooling steps indicated generally at 124. The heat recoveries 123 and 124 would in practice include for example (in decreasing order of heat grade)

pressured water heating (in a circuit including saturator 119)
shift feed heating (preliminary to exchanger 121)
saturator water heating

In addition 124 includes cooling to below the dewpoint of methanol, whereafter liquid crude methanol is separated in the lower section of catchpoint 126 and run off at 128 to distillative purification (not shown). Unreacted gas passing upwards in catchpot 126 is for the most part led out at 127 as a direct recycle stream, which is fed to an intermediate level in circulating pump 118. The rest of the unreacted gas enters the upper section of catchpot 126 via a chimney-plate and therein is freed of residual methanol vapour by contact on trays with cold water fed in at 125. Aqueous methanol is run off 131 to distillative purification. The scrubbed gas 129 is divided at 150. One stream from 150 is a wet purification stream ($CO_2$ removal), which is passed (path X) to absorption tower 144 and contacted therein with a regenerable solution such as potassium carbonate or physical solvent such as a dialkyl ether of polyethyleneglycol. (The regeneration means is not shown). (Dotted path Y is an alternative to path X and feeds the scrubbed gas to point 151 in purifier 12. Then the $CO_2$ removal step in purifier 12 replaces item 144. If desired, paths X and Y could both be used). The other stream from 150 is passed to dry purifier 152, which separates the first hydrogen stream, which is passed to mixing point 114, and a purge stream ($CH_4$, $CO_2$, $N_2$, CO, noble gases) at 153. Item 152 can be for example a cryogenic, adsorptive or diffusive unit. Stream 153 is the purge by which non-reactive gases are removed from the process.

Table 3 shows typical gas compositions and flow rates in the process, for producing 2345 metric tons of methanol per day.

In Figure 3 gasifier 10 and purifier 12 are as in Figure 2. The purified CO-rich gas from 12 is united at 215 with a hydrogen-rich stream to be described and passed to the inlet of circulating pump 218, which increases its pressure by about 10%. The total mixture has an $H_2$:CO ratio sufficient for methanol synthesis. Part of it is heated at 221 by indirect heat exchange with reacted synthesis gas to synthesis inlet temperature and passed into the first catalyst bed of reactor 222.

TABLE 3

| Position | Temp °C | Pressure at abs. | Gas composition % v/v | | | | | | Flow rate kg mol $h^{-1}$ |
|---|---|---|---|---|---|---|---|---|---|
| | | | CO | $CO_2$ | $H_2$ | $CH_4 + N_2$ | $CH_3OH$ | $H_2O$ | |
| 12 outlet | 35 | 38 | 67.05 | – | 31.22 | 1.74 | – | – | 11249 |
| 18 outlet | 35 | 38.5 | 34.45 | 10.83 | 51.00 | 3.09 | 0.61 | 0.02 | 70076 |
| 122 inlet | 210 | 38 | 32.90 | 10.34 | 48.71 | 2.95 | 0.58 | 4.52 | 73376 |
| 122 outlet | 240 | 37 | 28.76 | 14.48 | 52.85 | 2.95 | 0.58 | 0.37 | 73376 |
| E outlet | 270 | 36 | 26.89 | 15.73 | 48.42 | 3.22 | 5.24 | 0.50 | 67184 |
| 126 overhead | 35 | 35 | 28.33 | 16.49 | 51.01 | 3.39 | 0.74 | 0.03 | 63746 |
| 128 | 35 | 35 | 0.19 | 1.52 | 0.27 | 0.02 | – | – | 3437 |
| | | | | | | | – | 9.18 | 315.7 } liquid |
| | | | | | | | 88.82 | – | 3053.2 } liquid |
| 127 | 35 | 35 | 28.33 | 16.49 | 51.01 | 3.39 | 0.74 | 0.03 | 44623 |
| 150 – 144 | 35 | 35 | 28.54 | 16.61 | 51.39 | 3.42 | – | 0.03 | 13288 |
| 150 – 152 | 35 | 35 | 28.54 | 16.61 | 51.39 | 3.42 | – | 0.03 | 5695 |
| 144 – 115 | 35 | 35 | 33.55 | 2.02 | 60.41 | 4.01 | – | – | 11304 |
| 152 – 114 | 35 | 35 | – | 100 | – | – | – | – | 2634 |
| | 35 | 35 | 100 | – | – | – | – | – | 163 |
| 153 | 35 | 35 | 49.75 | 32.18 | 9.95 | 6.62 | – | 0.06 | 2898 |

The remainder, possibly after warming, is fed between the catalyst beds as quenches 223. The gas reacts in a first series of catalyst beds and is cooled between the beds by the quench gas feeds 223. After the first series of beds the gas is cooled by indirect heat exchange at 221 and finally reacted in the lowermost bed in reactor 222. It is then led out to heat recovery and cooling steps indicated generally by 224, which are the same as in the process of figure 2. Liquid methanol is separated in the lower section of catchpot 226 and run off at 228. Unreacted gas passing upwards in catchpot 226 is for the most part led off from the lower section as direct recycle stream 227, which is fed to an intermediate level in circulating pump 218. The rest enters the upper section of catchpot 226 via a chimney plate and therein is freed of residual methanol vapour by contact on trays with cold water fed in at 225. Aqueous methanol is run off at 231 to distillative purification. The scrubbed gas 229 receives an addition of steam by saturation with hot water at 230, is heated to shift inlet temperature at 231 and is passed into high temperature shift reactor 236. Here it reacts exothermally, whereafter it is cooled, by external heat recovery as *inter alia* hot water for saturator 230 and by heat exchange at 231 with feed gas, to the inlet temperature of low temperature shift reactor 237. Here the shift reaction is substantially completed. The resulting gas is cooled at 238 with external heat recovery and then to below the dewpoint of water, which is separated in catchpot 240 and run off at 242. The resulting water-depleted gas is contacted in 244 with a $CO_2$-absorbent solution fed in at 248 after being passed out at 246 to a regenerator (not shown). The $CO_2$-depleted gas is divided at 249 into a methanol stream (recycled to 215) and an ammonia stream, which is contacted at 252 with liquid nitrogen to give ammonia synthesis gas 253 and a discard stream 254. The ammonia synthesis section is conventional and is not shown.

The water fed to saturator 230 can include waste water from distillative purification of methanol and condensate from point 242, and is heated in a circuit including one or more of heat recoveries 224, 231 and 238 and others in items 10 and 12.

Table 4 shows typical gas compositions and flow rates in a process for producing per day 1823 metric tons of methanol and 650 of ammonia.

The following alternative forms of this process are to be noted:

(a) If desired, reactor 222 can be operated in the same way as reactor 122 of Figure 2, so as to effect shift reaction in its first bed;

(b) If gasifier 10 produced a gas containing more methane, it would be desirable to insert a methane/steam reaction step between points 230 and reactor 236. Heater 231 would then provide only external heat recovery. Heat would be recovered from the product of that reaction step when cooling it to the inlet temperature of reactor 222.

(c) If reaction systems 222—228 included aromatisation, then the alternative (b) will also be appropriate.

TABLE 4

| Position | Temp °C | Pressure at abs. | Gas composition % v/v | | | | | | Flow rate kg mol $h^{-1}$ |
|---|---|---|---|---|---|---|---|---|---|
| | | | CO | $CO_2$ | $H_2$ | $CH_4 + N_2$ | $CH_3OH$ | $H_2O$ | |
| 12 outlet | 35 | 35 | 64.36 | 4.00 | 29.97 | 1.67 | – | – | 10501 |
| 218 outlet | 35 | 38.5 | 32.20 | 3.06 | 59.10 | 5.19 | 0.39 | 0.06 | 41949 |
| 222 inlet | 240 | 38.00 | 32.20 | 3.06 | 59.10 | 5.19 | 0.39 | 0.06 | 41949 |
| 222 outlet | 270 | 36.00 | 29.85 | 3.43 | 53.59 | 5.89 | 7.13 | 0.11 | 37000 |
| 226 overhead | 35 | 35 | 31.93 | 3.65 | 57.34 | 6.30 | 0.77 | 0.01 | 34564 |
| 228 | 35 | 35 | 0.24 | 0.38 | 0.33 | 0.03 | – | – | 435 |
| | | | | | | | 97.45 | 1.57 | 2411 |
| 229 – 218 | 35 | 35 | 31.93 | 3.65 | 57.34 | 6.30 | 0.77 | 0.01 | 20739 |
| 236 outlet | 463.8 | 33 | 4.79 | 15.95 | 47.25 | 3.68 | – | 28.33 | 23720 |
| 237 outlet | 265.1 | 31.8 | 0.65 | 20.10 | 51.41 | 3.68 | – | 24.20 | 23720 |
| 244 outlet | 35 | 31.3 | 1.15 | 1.00 | 91.10 | 6.51 | – | 0.24 | 13380 |
| 248 – 215 | 35 | 31.3 | 1.15 | 1.00 | 91.10 | 6.51 | – | 0.24 | 10704 |
| 248 – 252 | 35 | 31.3 | 1.15 | 1.00 | 91.10 | 6.51 | – | 0.24 | 2676 |
| 253 | 35 | 30 | – | – | 100 | – | – | – | 2438 |

## Claims

1. A process for producing one or more carbon compounds by reaction of carbon monoxide (CO) with hydrogen which comprises the steps:

I providing a starting gas containing CO but deficient in hydrogen;
II processing it to produce a gas having the required hydrogen to CO ratio; and
III reacting the resulting gas incompletely over a catalyst to synthesise the said carbon compounds or an intermediate therefor;

and is characterised by

(i) producing the gas having the required hydrogen to CO ratio by mixing a hydrogen rich gas with the starting gas; and
(ii) producing the hydrogen rich gas by subjecting to the shift reaction and/or to the steam/hydrocarbon reaction and/or to carbon dioxide removal a mixture of the starting gas with either or both of (a) the product of such subjecting and (b) unreacted gas after separation of synthesis products in step III.

2. A process according to claim 1 in which the CO content of the starting gas is over 50% v/v and the CO content of the mixture is under 45% v/v.

3. A process according to claim 1 or claim 2 in which the mixture is subjected to a separate shift step at an outlet temperature in the range 300—550°C, followed by $CO_2$ removal.

4. A process according to claim 3 in which methanol is synthesised in step III and the shift reaction is controlled so as to give a methanol synthesis gas having a composition represented by

$$R = \frac{H_2 - CO_2}{CO + CO_2} = 1.0 \text{ to } 1.8$$

5. A process according to any one of the preceding claims in which steam is added to the gas entering the synthesis step III and shift reaction takes place over the synthesis catalyst or over a shift catalyst preceding the synthesis catalyst.

6. A process according to claim 1 in which the mixture contains a hydrocarbon or hydrocarbon derivative and is reacted with steam over a catalyst at an outlet temperature of at least 550°C and with a steam to hydrocarbon molar ratio of at least 2, whereby to convert the hydrocarbon to carbon oxides and hydrogen.

7. A process according to claim 6 in which the synthesis in step III is of methanol or dimethyl ether followed by aromatisation and the hydrocarbon is at least partly a by product of such aromatisation.

8. A process according to any one of the preceding claims in which the steam for the shift or steam/hydrocarbon reaction is introduced by contacting the gas mixture with hot water.

9. A process according to claim 8 in which the hot water is a waste water stream from the process and is heated by direct contacting with hot steam containing gas produced by the shift or steam/hydrocarbon reaction or by indirect heat exchange with reacted synthesis gas.

10. A process for producing methanol by reaction of CO with hydrogen which comprises the steps:

I providing a starting gas containing CO but deficient in hydrogen;
II processing it to produce a gas having the required hydrogen to CO ratio; and
III reacting the resulting gas incompletely over a copper containing catalyst to synthesise methanol;

and is characterised by:

(i) producing the gas having the required hydrogen to CO ratio by mixing a hydrogen rich gas with the starting gas; and
(ii) producing the hydrogen rich gas by subjecting to the shift reaction and/or to the steam/hydrocarbon reaction and/or to $CO_2$ removal one or a mixture of the following gases (a) a side stream of starting gas and (b) unreacted gas after separation of methanol in step III, and removing methane, noble gases and nitrogen from the gas to the extent necessary to attain less than 10% v/v of such gases in the gas to be fed to step III;
(iii) operating step III at a pressure in the range 20—50 bar abs, at a temperature under 300°C and at a space velocity in the range 5000—50000 $hour^{-1}$ and controlling these conditions to give methanol content of the reacted gas in the range 2—8% v/v, whereby the exothermic heat of synthesis is taken up by the gas in one or more adiabatic catalyst beds.

## Patentansprüche

1. Verfahren zur Herstellung von einer oder mehr als einer Kohlenstoffverbindung durch Umsetzung von Kohlenmonoxid (CO) mit Wasserstoff, das die folgenden Schritte enthält:

I Bereitstellung eines Ausgangsgases, das CO enthält, jedoch einen Wasserstoffmangel hat,

II Behandlung des Ausgangsgases zur Erzeugung eines Gases mit dem erforderlichen Wasserstoff/CO-Verhältnis und

III unvollständige Umsetzung des erhaltenen Gases über einem Katalysator zum Synthetisieren der Kohlenstoffverbindung(en) oder eines Zwischenprodukts dafür,
dadurch gekennzeichnet, daß

(i) das Gas mit dem erforderlichen Wasserstoff/CO-Verhältnis durch Vermischen eines wasserstoffreichen Gases mit dem Ausgangsgas erzeugt wird und

(ii) das wasserstoffreiche Gas erzeugt wird, indem eine Mischung des Ausgangsgases mit (a) und/oder (b) einer Behandlung in Form der katalytischen CO-Konvertierungsreaktion und/oder der Dampf/Kohlenwasserstoff-Reaktion und/oder einer Kohlendioxidentfernung unterzogen wird, wobei (a) das Produkt einer solchen Behandlung ist und (b) unumgesetztes Gas ist, das nach der Abtrennung der Syntheseprodukte im Schritt III erhalten wird.

2. Verfahren nach Anspruch 1, bei dem der CO-Gehalt des Ausgangsgases über 50 Vol.-% und der CO-Gehalt der Mischung unter 45 Vol.-% liegt.

3. Verfahren nach Anspruch 1 oder 2, bei dem die Mischung einem separaten katalytischen CO-Konvertierungsschritt bei einer Auslaßtemperatur im Bereich von 300 bis 550°C unterzogen wird, worauf eine $CO_2$-Entfernung folgt.

4. Verfahren nach Anspruch 3, bei dem im Schritt III Methanol synthetisiert und die katalytische CO-Konvertierungsreaktion so reguliert wird, daß sie ein Methanolsynthesegas mit einer durch

$$R = \frac{H_2 - CO_2}{CO + CO_2} = 1{,}0 \text{ bis } 1{,}8$$

ausgedrückten Zusammensetzung liefert.

5. Verfahren nach einem der vorhergehenden Ansprüche, bei dem zu dem Gas, das in den Syntheseschritt III eintritt, Dampf zugegeben wird und die katalytische CO-Konvertierungsreaktion über dem Synthesekatalysator oder über einem dem Synthesekatalysator vorausgehenden CO-Konvertierungs-Katalysator stattfindet.

6. Verfahren nach Anspruch 1, bei dem die Mischung einen Kohlenwasserstoff oder ein Kohlenwasserstoffderivat enthält und über einem Katalysator bei einer Auslaßtemperatur von mindestens 550°C und mit einem Dampf/Kohlenwasserstoff-Molverhältnis von mindestens 2 mit Dampf umgesetzt wird, um den Kohlenwasserstoff dadurch in Kohlenstoffoxide und Wasserstoff umzuwandeln.

7. Verfahren nach Anspruch 6, bei dem im Schritt III Methanol oder Dimethylether synthetisiert wird, worauf eine Aromatisierung folgt, wobei der Kohlenwasserstoff mindestens zum Teil ein Nebenprodukt einer solchen Aromatisierung ist.

8. Verfahren nach einem der vorhergehenden Ansprüche, bei dem der Dampf für die katalytische CO-Konvertierungsreaktion oder die Dampf/Kohlenwasserstoff-Reaktion eingeführt wird, indem die Gasmischung mit heißem Wasser in Kontakt gebracht wird.

9. Verfahren nach Anspruch 8, bei dem das heiße Wasser ein Abwasserstrom aus dem Verfahren ist und durch direkte Kontaktbehandlung mit heißen Dampf ent haltendem, durch die katalytische CO-Konvertierungsreaktion oder die Dampf/Kohlenwasserstoff-Reaktion erzeugtem Gas oder durch indirekten Wärmeaustausch mit umgesetztem Synthesegas erhitzt wird.

10. Verfahren zur Herstellung von Methanol durch Umsetzung von CO mit Wasserstoff, das die folgenden Schritte enthält:

I Bereitstellung eines Ausgangsgases, das CO enthält, jedoch einen Wasserstoffmangel hat,

II Behandlung des Ausgangsgases zur Erzeugung eines Gases mit dem erforderlichen Wasserstoff/CO-Verhältnis und

III unvollständige Umsetzung des erhaltenen Gases über einem kupferhaltigen Katalysator zum Synthetisieren von Methanol, dadurch gekennzeichnet, daß

(i) das Gas mit dem erforderlichen Wasserstoff/CO-Verhältnis durch Vermischen eines wasserstoffreichen Gases mit dem Ausgangsgas erzeugt wird,

(ii) das wasserstoffreiche Gas erzeugt wird, indem eines oder eine Mischung der folgenden Gase:

(a) eines Seitenstromes des Ausgangsgases und

(b) unumgesetzten Gases, das nach der Abtrennung von Methanol im Schritt III erhalten wird, der katalytischen CO-Konvertierungsreaktion und/oder der Dampf/Kohlenwasserstoff-Reaktion und/oder einer $CO_2$-Entfernung unterzogen wird und Methan, Edelgase und Stickstoff aus dem Gas in dem Ausmaß entfernt werden, das erforderlich ist, um in dem Gas, das dem Schritt II zuzuführen ist, einen Anteil solcher Gase von weniger als 10 Vol.-% zu erreichen, und

(iii) der Schritt III bei einem Druck im Bereich von 20 bis 50 bar abs., bei einer Temperatur unter 300°C und mit einer Raumgeschwindigkeit im Bereich von 5000 bis 50.000 $h^{-1}$ durchgeführt wird und diese Bedingungen so reguliert werden, daß ein im Bereich von 2 bis 8 Vol.-% liegender Methanolgehalt des umgesetzten Gases erhalten wird, wodurch die exotherme Synthesewärme von dem Gas in einem oder mehr als einem adiabatischen Katalysatorbett aufgenommen wird.

**Revendications**

1. Procédé de production d'un ou plusieurs composés du carbone par réaction du monoxyde de carbone (CO) avec l'hydrogène qui comprend les stades:
I d'utiliser un gaz de départ contenant du CO, mais déficient en hydrogène;
II de le traiter pour obtenir un gaz présentant le rapport requis de l'hydrogène au CO, et
III de faire réagir le gaz résultant incomplètement sur un catalyseur pour synthétiser ces composés du carbone ou un intermédiaire pour ceux-ci;
et est caractérisé en ce que
(i) on produit le gaz présentant le rapport requis de l'hydrogène au CO en mélangeant un gaz riche en hydrogène avec le gaz de départ, et
(ii) on produit le gaz riche en hydrogène par l'action de soumettre à la réaction de conversion et/ou à la réaction vapeur d'eau/hydrocarbure et/ou à l'élimination du dioxyde de carbone, un mélange du gaz de départ avec l'un et/ou l'autre de (a) le produit de l'action de soumettre et (b) le gaz qui n'a pas réagi après séparation des produits de synthèse au stade III.

2. Procédé suivant la revendication 1, dans lequel la teneur en CO du gaz de départ est supérieure à 50% v/v et la teneur en CO du mélange est inférieure à 45% v/v.

3. Procédé suivant la revendication 1 ou 2, dans lequel le mélange est soumis à un stade de conversion distinct à une température de sortie de l'intervalle de 300—550°C, suivi d'une élimination du $CO_2$.

4. Procédé suivant la revendication 3, dans lequel du méthanol est synthésisé au stade III et la réaction de conversion est conduite de manière à donner un gaz de synthèse du méthanol ayant une composition représenté par

$$R = \frac{H_2 - CO_2}{CO + CO_2} = 1{,}0 \text{ à } 1{,}8$$

5. Procédé suivant l'une quelconque des revendications précédentes, dans lequel de la vapeur d'eau est ajoutée au gaz entrant au stade de synthèse III at la réaction de conversion a lieu sur le catalyseur de synthèse ou sur un catalyseur de conversion précédant le catalyseur de synthèse.

6. Procédé suivant la revendication 1, dans lequel le mélange contient un hydrocarbure ou dérivé d'hydrocarbure et est mis à réagir avec de la vapeur d'eau sur un catalyseur à une température de sortie d'au moins 550°C et avec un rapport molaire de la vapeur d'eau à l'hydrocarbure d'au moins 2, de manière à convertir l'hydrocarbure en oxydes de carbone et hydrogène.

7. Procédé suivant la revendication 6, dans lequel la synthèse au stade III est celle du méthanol ou de l'éther diméthylique, suivie d'une aromatisation et l'hydrocarbure est au moins pour partie un sous-produit de cette aromatisation.

8. Procédé suivant l'une quelconque des revendications précédentes, dans lequel la vapeur d'eau pour la réaction de conversion ou vapeur d'eau/hydrocarbure est introduite par mise en contact du mélange gazeux avec de l'eau chaude.

9. Procédé suivant la revendication 8, dans lequel l'eau chaude est un courant d'eau usée provenant du procédé et est chauffée par contact direct avec du gaz chaud contenant de la vapeur d'eau produit par la réaction de conversion ou vapeur d'eau/hydrocarbure ou bien par échange de chaleur indirect avec du gaz de synthèse qui a réagi.

10. Procédé de production du méthanol par réaction du CO avec l'hydrogène qui comprend les stades:
I d'utiliser un gas de départ contenant du CO, mais déficient en hydrogène;
II de le traiter pour obtenir un gaz présentant le rapport requis de l'hydrogène au CO, et
III de faire réagir le gaz résultant incomplètement sur un catalyseur contenant du cuivre pour la synthèse du méthanol
et est caractérisé en ce que
i) on produit le gaz présentant le rapport requis de l'hydrogène au CO en mélangeant un gaz riche en hydrogène avec le gaz de départ;
ii) on produit le gaz riche en hydrogène par l'action de soumettre à la réaction de conversion et/ou à la réaction vapeur d'eau/hydrocarbure et/ou à l'élimination du $CO_2$ l'un des gaz suivants ou un mélange de ceux-ci (a) un courant prélevé sur le gaz de départ et (b) du gaz qui n'a pas réagi après séparation du méthanol au stade III, et on élimine le méthane, les gaz nobles et l'azote du gaz dans la mesure nécessaire pour arriver à moins de 10% v/v de ces gaz le gaz à admettre au stade III, et
iii) on exécute le stade III sous une pression de l'intervalle de 20—50 bars abs. à une température inférieure à 300°C et à une vitesse spatiale de l'intervalle de 5.000—50.000 $h^{-1}$ et on ajuste ces conditions pour établir une teneur en méthanol dans le gaz qui a réagi le l'intervalle de 2—8% v/v, de manière que la chaleur exothermique de la synthèse soit évacuée par le gaz dans un ou plusieurs lits de catalyseur adiabatiques.

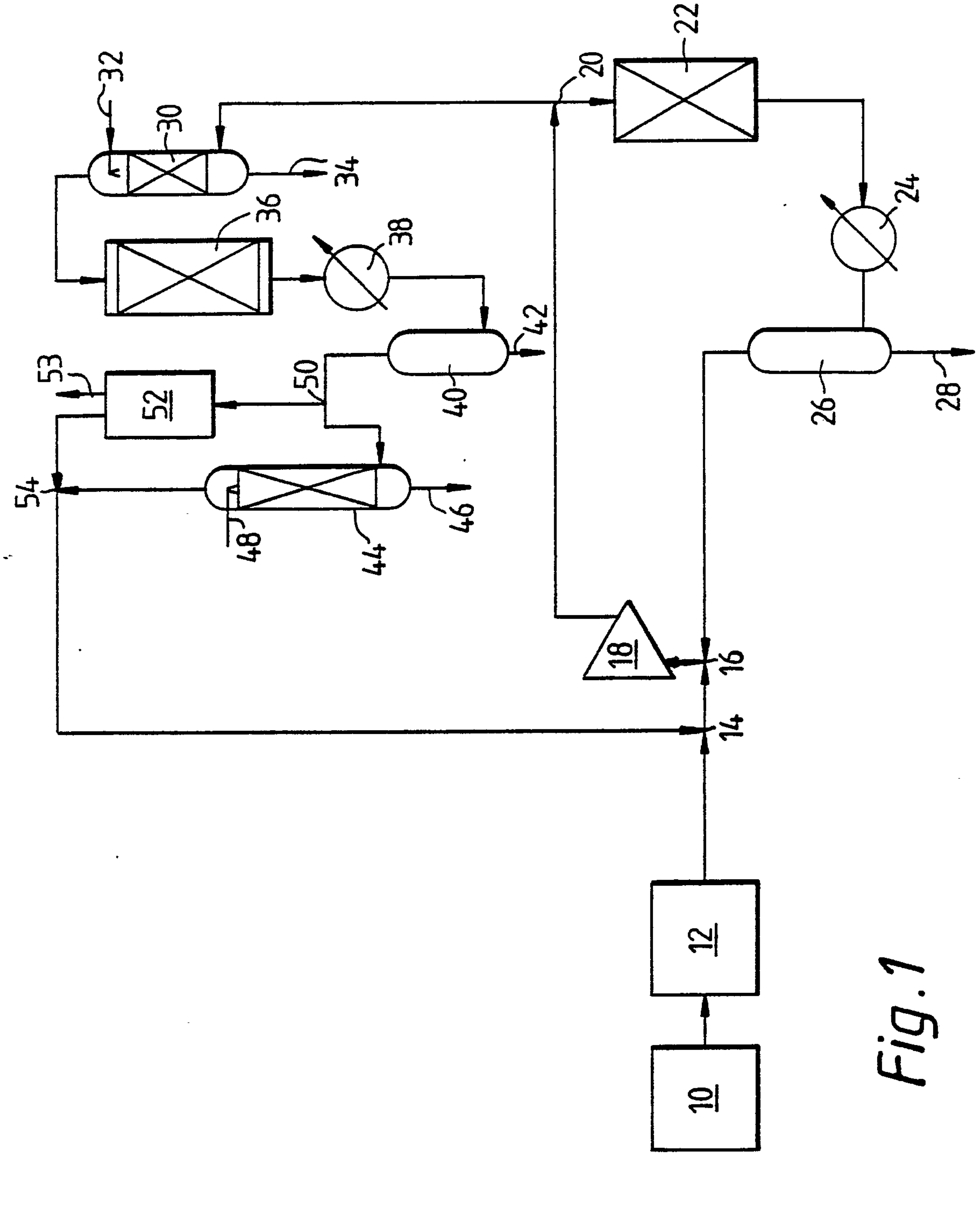


Fig. 1

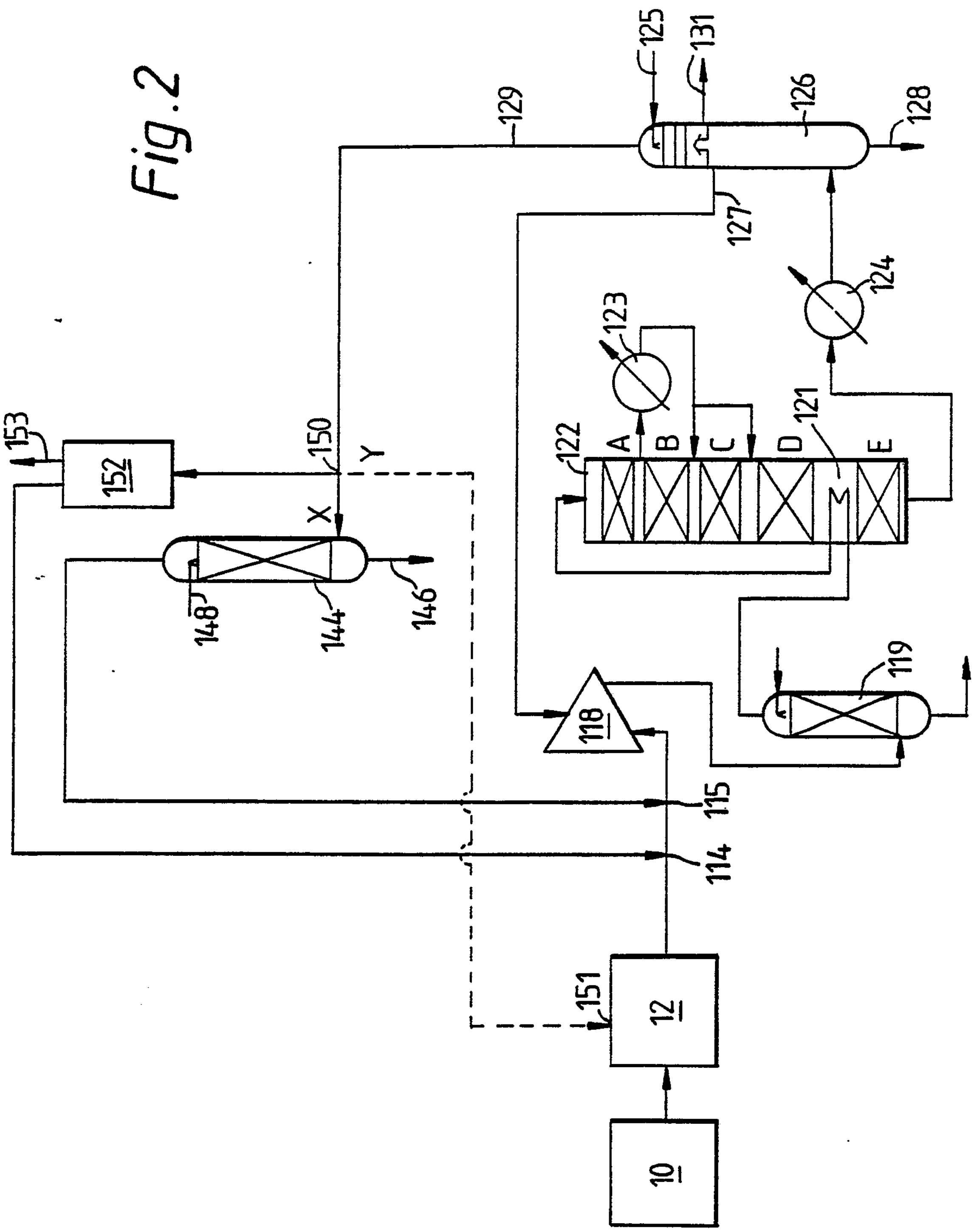
10
12
151
114
115
118
119
121
122
A
B
C
D
E
123
124
125
126
127
128
129
131
144
146
148
150
X
Y
152
153

Fig. 2

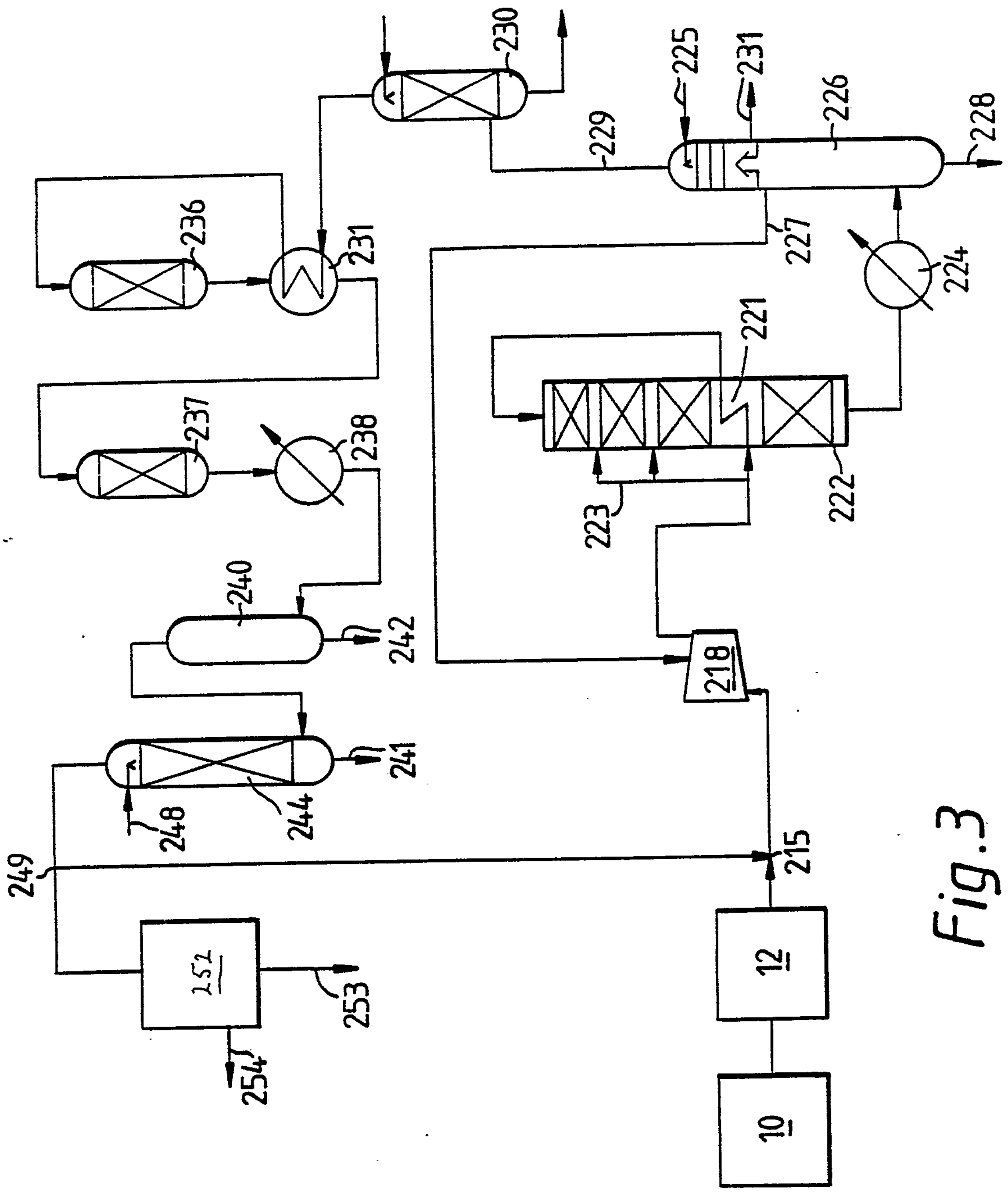

10
12
152
215
218
221
222
223
224
225
226
227
228
229
230
231
236
237
238
240
241
242
244
248
249
253
254


Fig. 3